## Europäisches Patentamt
## European Patent Office
## Office européen des brevets

(19)

(11) Veröffentlichungsnummer: **0 401 844 B1**

# EUROPÄISCHE PATENTSCHRIFT

(45) Veröffentlichungstag der Patentschrift: **22.11.95**

(51) Int. Cl.⁶: **A61L 31/00**

(21) Anmeldenummer: **90110847.2**

(22) Anmeldetag: **08.06.90**

(54) **Resorbierbare Formkörper und Verfahren zu ihrer Herstellung.**

(30) Priorität: **09.06.89 DE 3918861**

(43) Veröffentlichungstag der Anmeldung:
**12.12.90 Patentblatt 90/50**

(45) Bekanntmachung des Hinweises auf die
Patenterteilung:
**22.11.95 Patentblatt 95/47**

(84) Benannte Vertragsstaaten:
**AT BE CH DE DK ES FR GB GR IT LI LU NL SE**

(56) Entgegenhaltungen:
**EP-A- 0 107 591**
**EP-A- 0 185 453**
**EP-A- 0 209 371**
**EP-A- 0 349 656**
**US-A- 4 744 365**

(73) Patentinhaber: **BOEHRINGER INGELHEIM KG**
**Postfach 200**
**D-55216 Ingelheim (DE)**

(84) Benannte Vertragsstaaten:
**BE CH DE DK ES FR GR IT LI LU NL SE AT**

(73) Patentinhaber: **AESCULAP AG**
**Postfach 40**
**D-78501 Tuttlingen (DE)**

(84) Benannte Vertragsstaaten:

**BE CH DE DK ES FR GB GR IT LI LU NL SE AT**

(73) Patentinhaber: **BOEHRINGER INGELHEIM IN-**
**TERNATIONAL GmbH**
**Postfach 200**
**D-55216 Ingelheim am Rhein (DE)**

(84) Benannte Vertragsstaaten:
**GB**

(72) Erfinder: **Hurst, Achim**
**Föhrenstrasse 54**
**D-7200 Tuttlingen (DE)**
Erfinder: **Winkler-Gniewek,Wladis,Dr.Ing.**
**Regerweg 3**
**D-7200 Tuttlingen (DE)**
Erfinder: **Buchholz, Berthold, Dr.**
**Grundstrasse 55**
**D-6507 Ingelheim am Rhein (DE)**
Erfinder: **Bendix,Dieter,Dr.Dipl.-Chem.**
**Veith-Stoss-Strasse 17**
**D-6507 Ingelheim am Rhein (DE)**
Erfinder: **Entenmann,Günther,Dr.Dipl.-Chem.**
**Schützenpfad 16**
**D-6507 Ingelheim am Rhein (DE)**

EP 0 401 844 B1

## Beschreibung

Gegenstand dieser Erfindung sind resorbierbare Formkörper (Implantate) sowie die zur Herstellung dieser Formkörper (Implantate) verwendeten Stoffe bzw. Stoffgemische und Verfahren.

Zur Behandlung von Frakturen haben sich interne matallische Fixationssysteme weltweit durchgesetzt. Durch die Plattenosteosynthese können die Knochenfragmente immobilisiert werden. Ziel dieser Technik ist es, eine ungestörte Frakturheilung zu gewährleisten.

Der Einsatz von Metallimplantaten bringt aber auch Nachteile mit sich. Um Korrosion und Fremdkörperreaktionen zu vermeiden, müssen die Metallteile in einer zweiten Operation nach etwa einem Jahr wieder entfernt werden. Bei der Plattenosteosynthese verhindert die rigide Metallplatte remodellierende Umbauvorgänge im Bereich der Fraktur. Folge ist eine Inaktivitätsatrophie des Knochens, die nach der Entfernung der Implantate zur erneuten Fraktur führen kann. Darüberhinaus erschweren Metallimplantate die radiologische Verfolgung der Frakturheilung.

Zur Überwindung der Nachteile von Metallimplantaten wurde vielfach vorgeschlagen, die Implantate aus resorbierbaren Werkstoffen herzustellen. Solche Werkstoffe zeichnen sich dadurch aus, daß sie biologisch abbaubar sind. Trotz des, verglichen mit den Metallen, geringeren Elastizitätsmoduls eignen sich Implantate aus resorbierbaren Werkstoffen für die Knochenbruchbehandlung.

Voraussetzung ist, daß die mechanischen Eigenschaften auf die jeweiligen indikationsbezogenen Anforderungen abgestimmt sind. Infolge der Resorbierbarkeit des Materials bleiben den Patienten eine zweite Operation, die damit verbundenen Risiken und ein weiterer Arbeitsausfall erspart. Eine breite Einführung resorbierbarer Implantate in der Chirurgie wird deshalb zu einer beträchtlichen Kostenminderung führen.

Die Anforderungen an resorbierbare Osteosyntheseimplantate sind vielfältig. Grundvoraussetzungen für den medizinischen Einsatz resorbierbarer Werkstoffe sind die gute Gewebeverträglichkeit, die toxikologische Unbedenklichkeit der Polymeren und der Abbauprodukte und die Sterilisierbarkeit der Implantate. Neben adäquater Steifigkeit sollten die Implantate auch plastisch verformbar sein. Die Bruchdehnung sollte mindestens 2 %, bevorzugt mindestens 3 % betragen, um zum Beispiel Osteosyntheseplatten im Operationssaal an die individuelle Knochenform anpassen zu können. Diese Mindestdehnung gewährleistet außerdem ausreichende Sicherheit gegen Sprödbruch vor Erreichen der Fließgrenze. Besonders bruchgefährdet sind Fixationselemente, an denen Bohrungen (z.B. Osteosyntheseplatten) oder größere Querschnittsänderungen (z.B. Schrauben) auftreten. Durch Kerbwirkung können Spannungszustände und -überhöhungen entstehen, denen ein spröder Werkstoff nicht durch Verformungsarbeit widerstehen kann.

Es sind zahlreiche Werkstoffe bekannt, welche im Organismus abgebaut werden können. Unter diesen Werkstoffen haben Polymere und Copolymere der Milchsäuren und der Glycolsäure wegen ihrer bekannten Verträglichkeit besondere Bedeutung erlangt.

So sind chirurgrische Gegenstände, insbesondere Osteosyntheseplatten, Schrauben und andere Befestigungselemente aus derartigen Polymeren in zahlreichen Publikationen beschrieben:

Beispielsweise offenbart die Europäische Offenlegungsschritt EP-A-0 107 591 absorbierbare; im wesentlichen amorphe chirurgische Befestigungselemente, die aus Copolymerisaten aus 70-85 Mol-% Laktid und 15-30 Mol-% Glykolid herstellbar sind, wobei das Befestigungselement aus einem Copolymerisat mit einer Glasumwandlungstemperatur u.a. von mindestens 56°C und einer Eigenviskosität von mindestens 13 dl/g hergestellt wird. Die Herstellung selbst kann auf dem Wege des Spritzgießens erfolgen. - Die Aufgabe, die dieser Entgegenhaltung zugrundeliegt, besteht darin, Befestigungselemente mit Materialcharakteristika zur Verfügung zu stellen, die eine genügend große Festigkeit über einen bestimmten Implantationszeitraum beibehalten, und die aus einem im wesentlichen amorphen Copolymerisat aufgebaut werden.

Der Erfindung dieser Offenlegungsschritt liegt die Erkenntnis zugrunde, daß - entgegen zu der zum Anmeldezeitpunkt in der Fachwelt vertretenen Auffassung - nicht nur kristalline sondern auch Befestigungselemente aus amorphen Laktid/Glykolid - Copolymeren die für Befestigungselemente erforderlichen mechanischen Eigenschaften aufweisen.

Die Europäische Offenlegungsschrift EP-A-0 185 453 hat ebenfalls chirurgische Befestigungselemente zum Gegenstand, die aus sog. "Polymerblends" aus einem Laktid/Glykolid-Copolymerisat und Poly(p-dioxanon) hergestellt werden. Die Aufgabe, die dieser Erfindung zugrundeliegt, besteht darin, eine chirurgische Befestigungseinheit zur Verfügung zu stellen, die neben befriedigenden mechanischen Eigenschaften auch eine gute thermische Belastbarkeit aufweist.

Daneben wird in dieser Offenlegungsschrift offenbart, daß die einzelnen Bestandteile des Befestigungselementes mittels Spritzguß hergestellt werden können, wobei die Temperatur in einem Bereich von 130 bis 140°C liegt. Dabei werden die beiden Polymere - Laktid/Glykolid-Copolymer und Poly-[p-dioxanon] z.B. in Pelletform in der Spritzgießapparatur vorgelegt.

Eine weitere europäische Offenlegungsschrift - die EP-A-0 209 371 - betrifft ebenfalls ein chirurgisches Befestigungselement, das aus einem Glykolid-reichen Polymerblend hergestellt wird. Aufgabe dieser Erfindung ist es - ähnlich zum vorgenannten Dokument - ein chirurgisches Befestigungselement zur Verfügung zu stellen, das im Rahmen des Spritzgußverfahrens herstellbar ist und das über einen ausreichend langen Implantationszeitraum eine ausreichende mechanische Stabilität behält.

Die US-amerikanische Patentschrift US-A-4 744 365 hat ebenfalls ein chirurgisches Befestigungselement zum Gegenstand, das aus einer sog. "multi-phase" Polymerzusammensetzung hergestellt wird. Dabei weist - beispielsweise in einer zweiphasigen Zusammensetzung - die erste Phase 0 bis ca. 25 Mol-% Glykolideinheiten. Die Zusammensetzung kann dabei insgesamt bis zu 45 Mol-% Glykolid enthalten, wobei die erste Phase mindestens 50 % aber - vorzugsweise - nicht mehr als 95 % der gesamten Zusammensetzung ausmacht!

Die Vorrichtungen aus derartigen mehrphasischen Polymerzusammensetzungen besitzen eine hohe Verformungstemperatur und können ebenfalls auf dem Wege des Spritzgießverfahrens hergestellt werden. Die dem Stand der Technik entsprechenden Ausführungsformen weisen jedoch zahlreiche Nachteile auf:

In der EP 0108635 ist beispielsweise dargelegt, daß sich zur Herstellung chirurgischer Gegenstände vorzugsweise Poly(L-lactid) mit hoher inhärenter Viskosität eignet. Hierzu wird L-Lactid unter genau einzuhaltenden Bedingungen, insbesondere mit sehr langen Reaktionszeiten, polymerisiert. Die aus den Polymerisaten durch mechanische Bearbeitung erhaltenen Probekörper weisen bei einer inhärenten Viskosität von 7,4 dl/g eine Zugfestigkeit von 58,2 N/mm$^2$ auf. Bei einer inhärenten Viskosität von 3,7 dl/g beträgt die Festigkeit nur 28,8 N/mm$^2$. Außerdem weisen die so hergestellten Gegenstände eine mikroporöse Struktur auf (vgl. J. W. Leenslag, A. J. Pennings Commun. 28 92-94 (1987), Makromol. Chem. 188, 1809-1814 (1987)). Dadurch wird das Eindringen von Wasser erleichtert und der Abbau sowie der Verlust der mechanischen Festigkeit beschleunigt. So beträgt die Zugfestigkeit der oben beschriebenen Polymeren nach 12 Wochen bei 37 °C in Pufferlösung nur noch 13 - 16 % des Ausgangswertes. Aus den gemäß EP 0108635 hergestellten Polymerisaten sind chirurgische Gegenstände durch spanabhebende Verfahren hergestellt worden. Bei der Bearbeitung treten an der Oberfläche der Gegenstände Riefen und andere Schäden auf, welche bei statischer oder dynamischer Belastung und insbesondere bei schlagartiger Beanspruchung zu Rissen und folglich zum Versagen der Teile führen können.

Im Verlauf des hydrolytischen Abbaus von chirurgischen Gegenständen nimmt die Festigkeit bekanntermaßen ab.

Bei mikroporösem Material, gemäß EP 0108635, erfolgt die Festigkeitsabnahme besonders rasch (vgl. z.B. Eitenmüller et al., Chirurg 58, 831-839 (1987)).

Man unterscheidet verschiedene Arten von Dehnungen. Charakteristisches Maß für die Zähigkeit und die bleibende Verformbarkeit eines Werkstoffs ist die Bruchdehnung. Die Bruchdehnung ist die auf eine Anfangsmeßlänge bezogene bleibende Längenänderung nach dem Bruch einer Zugprobe.

Poly(L-lactid) zeigt bei Beanspruchung sprödes Verhalten. Die Bruchdehnung beträgt nur ca. 2 % (M. Vert et al., Makromol. Chem. Suppl. 5, 30-41 (1981)). Bei schnellen Belastungswechseln besteht deshalb die Gefahr des Bruchs der Implantate. Copolymere des L-Lactids weisen eine höhere Bruchdehnung als das reine Poly(L-lactid) auf. Allerdings nimmt mit zunehmendem Comonomeranteil die Festigkeit bekanntermaßen ab (vgl. z.B. US-PS 3736646). Vert et al. (Macromol. Biomat. 1984, 119 - 142) geben für Poly(L-lactid) eine Zugfestigkeit von 58 N/mm$^2$ bei einer Bruchdehnung von 2,1 %, für Poly(L-lactid-co-D,L-lactid) 50 : 50 bei einer Zugfestigkeit von 46 N/mm$^2$ eine Bruchdehnung von 3,2 % an, Formkörper dieser Zusammensetzung weisen jedoch den gravierenden Nachteil auf, daß ihre ursprüngliche Festigkeit bereits innerhalb von zwei Wochen auf die Hälfte des ursprünglichen Wertes gefallen ist.

Die Zugabe von Comonomeren bei der Polymerisation erhöht somit zwar die Bruchdehnung, verringert aber gleichzeitig die Anfangsfestigkeit und die Festigkeitsretention in unerwünschter Weise. Diese Nachteile haben zu dem Vorschlag geführt (Vert et al., Macromolecular Biomaterials 1984), chirurgische Gegenstände aus vollständig resorbierbaren faserverstärkten Verbundwerkstoffen herzustellen. Solche faserverstärkten Verbundwerkstoffe und ihre Herstellung sind z.B. in WO 88/00533, WO 87/00059 oder EP 0011528 beschrieben. Auch die Verwendung von nicht resorbierbaren Verstärkungselementen wurde z.B. in der US-PS 4329743 vorgeschlagen. Die Herstellung von faserverstärkten Gegenständen ist jedoch technisch aufwendig. Auch lassen sich auf diese Weise nur einfache Formkörper, vorzugsweise Stifte, herstellen.

Es ist die Aufgabe der vorliegenden Erfindung, einen resorbierbaren Formkörper (Implantat) zur Verfügung zu stellen, der neben hoher Anfangsbiegefestigkeit und hoher Anfangszugfestigkeit auch hohe Festigkeitsretention und optimale Bruchdehnung aufweist, jedoch technisch einfacher herstellbar ist als faserverstärkte Implantate. Resorbierbare Implantate, die diese Anforderungen erfüllen, sollten sich neben ausreichender initialer Stabilität durch die Erhaltung der Festigkeit während der Dauer der Frakturheilung, das entspricht einem Zeitraum von ca. 6 - 8 Wochen nach der Operation, auszeichnen, wobei die Festigkeit

EP 0 401 844 B1

8 Wochen nach der Implantation noch mindestens 75 % des Ausgangswertes bei einer Bruchdehnung von mehr als 2,1 % betragen soll. Während dieser Phase übertragen die Implantate die auftretenden Kräfte und immobilisieren die Knochenfragmente. Nach erfolgter Einheilung verlieren die Werkstoffe bedingt durch den biologischen Abbau allmählich ihre Festigkeit. Die zunehmende Belastung führt zu einer funktionellen Strukturierung des Knochens im Frakturspalt durch eine der biomechanischen Beanspruchung entsprechende Ausrichtung der Knochenbälkchen. Die Knochenatrophie durch unphysiologische Stützwirkung des Implantates wird dadurch verhindert.

Erfindungsgemäß wird die Aufgabe durch einen Formkörper auf der Basis von Poly(lactid) oder davon abgeleiteten Copolymerisaten gelöst, der einen hochmolekularen Zusatzstoff aus einem resorbierbaren Polyester in einer wirksamen Menge zur Erhöhung der Zugfestigkeit und der Bruchdehnung enthält, wobei der Glycolidgehalt der Zusammensetzung weniger als 65 Gew.-% beträgt und Poly[p-dioxanon] als Zusatzstoff ausgenommen ist. Der erfindungsgemäße Formkörper ist dabei bevorzugt auf dem Wege des Spritzgußverfahrens herstellbar.

Mischungen aus Polylactid sowie dessen Copolymeren mit monomeren oder polymeren Zusatzstoffen werden auch als Polymermischungen oder "Polymerblends" bezeichnet. - Erfindungsgemäß sind die zuvorgenannten Formkörper, die Polymermischungen enthalten, bevorzugt.

Des weiteren sind Formkörper bevorzugt, die aus Poly(L-lactid), Poly(D-lactid) oder davon abgeleiteten Copolymerisaten mit anderen Comonomeren in Form copolymerisierbarer cyclischer Ester bestehen, wobei der Comonomeranteil nicht mehr als 30 Gew.-% - bevorzugt nicht mehr als 15 Gew.-% - beträgt.

Die zuvorgenannte Aufgabe wird zudem bevorzugt durch Formkörper, die aus einem Copolymerisat aufgebaut sind, gelöst, wobei das Comonomer D,L-Lactid, meso-Lactid, Glycolid, Trimethylencarbonat oder ein Lacton der $\beta$-Hydroxybuttersäureund/oder $\beta$-Hydroxyvaleriansäure ist.

Formkörper gemäß dieser Erfindung sind Implantate für alle Bereiche der Chirurgie. In der Knochenchirurgie können beispielsweise Osteosyntheseplatten zusammen mit Verbindungselementen wie Schrauben, Spreizdübeln oder Nieten zur Vereinigung und temporären Fixierung von Knochenfragenmenten eingesetzt werden. Glatte oder profilierte Fixationsstifte, Fixationsnägel oder Schrauben eignen sich zur Refixation von Knorpel- oder Knochenfragmenten. Frakturen von Röhrenknochen lassen sich mit Markraumnägeln intramedullär solange stützen, bis die Frakturheilung abgeschlossen ist. Auch Clips zum Verschluß von Gefäßen oder Klammern zum Vernähen von Weichgewebe sind beispielhafte Gegenstände aus den erfindungsgemäßen Werkstoffen. Die vorteilhafte Bruchdehnung, die günstige Festigkeit und die Festigkeitsretention während der Heilungsphase des erfindungsgemäßen Implantats gewährleisten den Behandlungserfolg. Es versteht sich von selbst, daß kunststoffgerechtes Konstruieren zu einer Vielzahl von Ausführungsformen dieser Gegenstände führt. Diese Aufzählung ist daher beispielhaft und keinesfalls einschränkend zu verstehen.

Spritzgegossene Formkörper gemäß dieser Erfindung weisen auch bei komplizierter Bauweise glatte Oberflächen und im Gegensatz zu Formkörpern, welche durch mechanische Bearbeitung aus Polymerblökken erhalten werden, keine mikroporöse Struktur auf. Folglich kann Kerbwirkung durch Mikrofehler verhindert werden.

Außerdem liegt eine signifikant bessere Festigkeitsretention der erfindungsgemäßen spritzgegossener Proben vor: In Tierversuchen konnte nachgewiesen werden, daß die Festigkeit implantierter Probekörper aus Poly(L-lactid)-Blockware, die nicht durch Spritzguß hergestellt wurden, trotz hoher inhärenter Viskosität (i.V. 7,9 dl/g) bereits nach 8 Wochen auf 18,5 % der initialen Biegefestigkeit fiel.

Durch diesen hohen Festigkeitsverlust des Implantatwerkstoffs bereits innerhalb der Knochenheilungsphase kann der Erfolg der Operation gefährdet werden. Dagegen zeigen die erfindungsgemäßen spritzgegossene Proben aus Poly(L-lactid) trotz vergleichweise geringer inhärenter Viskosität (i.V. 1,65 dl/g) mit 97,0 % der initialen Festigkeit nach 8 Wochen die den Anforderungen entsprechende Festigkeitsretention. (Beispiel 1). Die zur Herstellung des erfindungsgemäßen Formkörpers eingesetzten Polymere, z.B. Polylactid, Copolymere oder die erfindungsgemäßen Polymermischungen weisen im allgemeinen eine inhärente Viskosität von kleiner 4,5 dl/g, bevorzugt kleiner 3,7 dl/g und besonders bevorzugt kleiner 3,0 dl/g auf, sollten jedoch nicht kleiner als 1,0 bevorzugt nicht kleiner als 1,4 dl/g sein.

Der Spritzgußprozeß führt je nach den Prozeßbedingungen zu mehr oder weniger starkem thermischen Abbau des Polymeren und damit zu einer Verringerung der inhärenten Viskosität. Es ist bekannt, daß Feuchtigkeit und ein Restgehalt an Monomeren den thermischen Abbau begünstigen. Es versteht sich daher von selbst, daß die Polymeren vor dem Einsatz durch an sich bekannte Verfahren wie Extraktion oder Umfällung und/oder Erhitzten im Vakuum sorgfältig zu reinigen und zu trocknen sind. Überraschenderweise wurde jedoch festgestellt, daß spritzgegossene Formkörper trotz verringerter inhärenter Viskosität ebenso hohe Anfangsfestigkeiten aufweisen wie Formkörper aus Polymerblöcken (Beispiel 2). Die Verwendung von Implantaten aus Poly(L-lactid) mit niedriger inhärenter Viskosität ist deshalb besonders vorteilhaft, weil sich

4

die Resorptionszeit im Vergleich zu hochmolekularem Material verkürzt: Mit in vitro-Versuchen bei erhöhter Temperatur in physiologischer Lösung konnte nachgewiesen werden, daß gespritzte Probekörper aus Poly-(L-lactid) (i.V. 1,65 dl/g) rascher hydrolysieren als Proben aus Poly(L-lactid)-Blockpolymer (i.V. 7,9 dl/g). Bei Implantaten aus hochviskoser Poly(L-lactid)-Blockware muß von Resorptionszeiten von mehr als 3 Jahren ausgegangen werden. Bei gespritzten Implantaten gleicher Größe und Festigkeit ist mit Resorptionszeiten zwischen 1,5 und 2,5 Jahren zu rechnen.

Somit ist ein thermischer Abbau von Polymeren beim Spritzguß in gewissem Ausmaß tolerierbar, d.h. spritzgegossene Polymere sind bezüglich des Reinigungsprozesses weniger kritisch als Blockware.

Gegenstand der Erfindung sind darüber hinaus sowohl amorphe wie kristalline spritzgegossene Formkörper. Formkörper aus Poly(L-lactid), welche aus Polymerblöcken erhalten werden, weisen je nach Polymerisationsbedingungen Kristallinitätsgrade von > 75 % auf. Die Kristallinität wird in bekannter Weise durch Bestimmung der Schmelzenthalpie mittels Differential-Scanning-Calorimetry (DSC) und Vergleich des Wertes mit der literaturbekannten Schmelzenthalpie von 100 % kristallinem Polylactid bestimmt. Im Spritzgußprozeß können in Abhängigkeit von der Verweilzeit der Formkörper im Werkzeug und der Abkühlgeschwindigkeit amorphe oder kristalline Produkte erhalten werden. In Beispiel 1 und 2 wurden amorphe spritzgegossene Formkörper hergestellt. Der Vergleich mit den kristallinen Probekörpern aus Polymerblöcken zeigt, daß die Kristallinität auf die Anfangsfestigkeit keinen Einfluß hat. Dagegen haben die kristallinen Produkte einen höheren E-Modul (Elastizitäts-Modul) als amorphe Formkörper. In weitergehenden Untersuchungen wurde außerdem festgestellt, daß sich amorphes Poly(L-lactid) im Abbauverhalten von kristallinem Poly(L-lactid) unterscheidet. Je nach Art des herzustellenden chirurgischen Gegenstandes kann es daher erforderlich sein, wahlweise amorphe oder kristalline Produkte durch Spritzguß herzustellen. Die Kristallinität kann wie oben beschrieben durch Wahl der Spritzbedingungen und/oder in an sich bekannter Weise durch den Zusatz von Nucleierungsmitteln beeinfluß werden. Im Rahmen dieser Erfindung kommen aus naheliegenden Gründen nur physiologisch unbedenkliche Nucleierungsmittel wie Salze verträglicher organischer Säuren wie Calciumcitrat oder hochschmelzende Polymere wie Polyglycolsäure in Frage. Die Aufzählung ist beispielhaft und nicht einschränkend zu verstehen. Kristalline Produkte können auch nachträglich aus amorphen Formkörpern durch Tempern erhalten werden. Im Falle von Poly(L-lactid) reicht hierfür einfaches Erwärmen für eine längere Zeit (mindestens 30 Minuten) auf höhere Temperatur (mehr als 70 °C) aus. Die genauen Temperungsbedingungen können gemäß dem gewünschten Kristallisationsgrad optimiert und eingestellt werden.

Unter bestimmten Bedingungen kommt es beim Spritzgußprozeß zu Orientierungen der Moleküle innerhalb des Spritzlinges, die sich festigkeitssteigernd auswirken. Im Extremfall führt dies zu einer für bestimmte Anwendungsgebiete erwünschten Anisotropie der mechanischen Eigenschaften, d.h. die Festigkeit eines Implantats ist z.B. in Längsrichtung höher als in Querrichtung. Die Festigkeit in Längsrichtung ist dabei höher als die Festigkeit in einem sonst gleichen isotropen Formkörper.

Alle bisher für spritzgegossene Formkörper aufgeführten Eigenschaften gelten auch für Gegenstände, welche mit anderen bei thermoplastischen Polymeren einsetzbaren Verarbeitungsschritten wie Extrusion, Druckschmelzen, Heißverpressen usw. hergestellt wurden. Der Ausdruck "Spritzguß" und spritzgegossen ist somit keinesfalls einschränkend zu verstehen. Die Erfindung betrifft vielmehr Gegenstände, welche in irgendeiner Weise durch thermoplastische Verformung hergestellt oder verändert wurden.

Polylactid gemäß dieser Erfindung ist Poly(L-lactid) und Poly(D-lactid), wobei jedoch Poly(L-lactid) bevorzugt wird. Ebenfalls Gegenstand dieser Erfindung sind Copolymere der beiden genannten Lactide mit Comonomeren, welche zu physiologisch unbedenklichen Abbauprodukten führen.

Solche Comonomeren sind D,L-Lactid, meso-Lactid, Glycolid, Dioxanon, Trimethylencarbonat und weitere mit Lactid copolymerisierbare cyclische Ester. Andere geeignete Comonomere sind $\alpha$-, $\beta$- oder $\gamma$-Hydroxybuttersäure, $\alpha$-, $\beta$- oder $\gamma$-Hydroxyvaleriansäure und andere Hydroxyfettsäuren ($C_{11}$ bis $C_{25}$) wie z.B. Stearinsäure, Palmitinsäure, Ölsäure, Laurinsäure u.a.

Bevorzugt werden jedoch D,L-Lactid, meso-Lactid, Glycolid, $\beta$-Hydroxybuttersäure $\beta$-Hydroxyvaleriansäure, besonders bevorzugt wird D,L-Lactid. Es hat sich gezeigt, daß der Zusatz von Comonomeren die Festigkeitswerte verschlechtert. Für die Auswahl des im Einzelfall geeigneten Copolymeren ist somit ein Kompromiß zwischen den verringerten Festigkeitswerten und verbesserten anderen Eigenschaften wie Bruchdehnung und Abbauverhalten zu schließen. Für die oben aufgeführten Anwendungsbereiche soll der Comonomeranteil nicht mehr als 30 %, vorzugsweise nicht mehr als 15 % betragen. Geeignet sind Copolymere des L-Lactids und des D-Lactids; bevorzugt werden Polymere des L-Lactids. Bevorzugt sind weiterhin Formkörper aus Poly(L-lactid-co-D,L-lactid mit inhärenten Viskositäten zwischen 1 und 3,5 dl/g, besonders bevorzugt zwischen 1,4 und 2,5 dl/g. Der Anteil an L-lactid in dem Copolymerisat beträgt zwischen 70 und 90% bevorzugt zwischen 75 und 85%.

In Beispiel 3 ist die Herstellung von spritzgegossenen Probekörpern aus Poly(L-lactid-co-D,L-lactid) 90 : 10 beschrieben. Im Vergleich zu Beispiel 2 A weist das Produkt eine vorteilhaft erhöhte Bruchdehnung und eine erhöhte Zugfestigkeit auf.

Gegenstand dieser Erfindung sind insbesondere Formkörper (Implantate) mit erhöhter Bruchdehnung. Es ist bekannt, daß die Bruchdehnung von Thermoplasten durch Zusatz von niedermolekularen Flüssigkeiten, niedermolekularen Feststoffen oder hochmolekularen Feststoffen erhöht werden kann (= Weichmachereffekt). Bei Zusatz von flüssigen Stoffen spricht man üblicherweise von Weichmachern, bei Zusatz polymerer Feststoffe von Polymerblends. Normalerweise (vtl. H.-G. Elias, Makromoleküle; Hüthig & Wepf, Basel 1981, S. 949) erhöhen Weichmacher die Kettenbeweglichkeit. Dies führt zwar zu einer Heraufsetzung der Bruchdehnung, gleichzeitig erniedrigen sich jedoch Glasübergangstemperatur, Elastizitätsmodul, Reißfestigkeit und Härte. Im vorliegenden Fall war somit durch den Zusatz von Weichmachern keine vorteilhafte Wirkung auf resorbierbare Implantate zu erwarten.

Überraschenderweise wurde jedoch gefunden, daß der Zusatz bestimmter als Weichmacher geeigneter Flüssigkeiten zu einer vergleichbaren Festigkeit bei gleichzeitig signifikanter Erhöhung der Bruchdehnung führt. Andere ebenfalls potentiell als Weichmacher geeignete Flüssigkeiten führten bei vergleichbarer Festigkeit nicht zu einer Erhöhung der Bruchdehnung (siehe Beispiel 4). Geeignete Weichmacher sind Acetyltributylcitrat und Glycerintriacetat sowie Gemische beider Komponenten.

Ein weiterer Gegenstand der vorliegenden Erfindungen sind Formkörper auf der Basis von resorbierbaren Polymermischungen, insbesondere auf der Basis von Polylactid, das als Zusatzstoff einen hochmolekularen Feststoff (einen Polyester) enthält. Als Polylactid gemäß dieser Erfindung wird Poly(L-lactid), Poly(D-lactid), Poly(meso-lactid) und Poly(D,L-lactid) bezeichnet, wobei Formkörper auf der Basis von Poly(L-lactid) bevorzugt sind.

In weiteren Versuchen wurde überraschenderweise festgestellt, daß der Zusatz von bestimmten hochmolekularen Feststoffen (erfindungsgemäß auch als Zusatzstoffe bezeichnet) zu Poly(L-lactid) nicht nur zu einer Erhöhung der Bruchdehnung, sondern auch zu einer signifikanten Erhöhung der Zugfestigkeit führt (Beispiel 5). Während die Erhöhung der Bruchdehnung durch Beimischen einer zäheren Komponente den Erwartungen entspricht, stellt die gleichzeitige vorteilhafte Erhöhung der Zugfestigkeit ein gänzlich unerwartetes Ergebnis dar. Besonders vorteilhaft ist, daß bei einem Formkörper (Implantat) aus Poly(L-lactid) welches erfindungsgemäß den Zusatzstoff (Feststoff) enthält mehr als 75 % der Zugfestigkeit und deutlich mehr als 3 % Bruchdehnung auch noch nach einem Zeitraum von 8 Wochen unter simulierten physiologischen Bedingungen (37 °C, Ringerlösung) erhalten bleiben. Entsprechend der Aufgabenstellung müssen die hochmolekularen Feststoffe zu physiologisch unbedenklichen Produkten abbaubar sein. Erfindungsgemäße Feststoffe sind resorbierbare Polyester, wie z.B. Poly(D,L-lactid), Poly(D-lactid), Poly(meso-lactid), Poly(glycolid), Poly(trimethylencarbonat), Poly(dioxanon), Poly(caprolacton) sowie die aus beliebigen Kombinationen von L-Lactid, D-Lactid, meso-Lactid, D,L-Lactid, Glycolid, Trimethylencarbonat, Dioxanon, Caprolacton und andere, dem Fachmann hinreichend bekannte aus polymerisationsfähigen cyclischen Estern herstellbare Co- und Terpolymere. Bevorzugte hochmolekulare Feststoffe sind Poly(D,L-lactid), Poly(meso-lactid), Poly(dioxanon) und Poly(caprolacton). Besonders bevorzugt werden Poly(D,L-lactid) und Poly(meso-lactid). Geeignete Copolymere sind beispielsweise

Poly(L-lactid-co-D,L-lactid)
Poly(L-lactid-co-meso-lactid)
Poly(L-lactid-co-glycolid)
Poly(L-lactid-co-trimethylencarbonat)
Poly(L-lactid-co-epsilon-caprolacton)
Poly(D,L-lactid-co-meso-lactid)
Poly(D,L-lactid-co-glycolid)
Poly(D,L-lactid-co-trimethylencarbonat)
Poly(D,L-lactid-co-epsilon-caprolacton)
Poly(meso-lactid-co-glycolid)
Poly(meso-lactid-co-trimethylencarbonat)
Poly(meso-lactid-co-epsilon-caprolacton)
Poly(glycolid-co-trimethylencarbonat)
Poly(glycolid-co-epsilon-caprolacton)

Die Menge an zugesetzten hochmolekularem Feststoff kann im allgemeinen 1 bis 50 % in Einzelfällen bis zu 85% betragen. Bevorzugt werden jedoch Zusätze zwischen 5 und 35 %, besonders bevorzugt 5 bis 25 %. Bei geringen Zusätzen ist die inhärente Viskosität des zugesetzten hochmolekularen Feststoffes nicht kritisch. Im allgemeinen liegt die Viskosität des Zusatzstoffes im Bereich der Viskosität des Basispolymers, wie sie zuvor definiert wurde. Bei Zusätzen von 10 % oder mehr sollte sie jedoch mehr als 1 dl/g (25 °C, Chloroform) betragen. Werden Copolymere eingesetzt, ist deren Sequenz nicht kritisch. Statistische

6

Copolymere und Block-Copolymere sind gleichermaßen geeignet.

Bevorzugtes Copolymer ist Poly(L-lactid-co-D,L-lactid).

Copolymere die L-Lactid als Komponente enthalten, enthalten bevorzugt mindestens 70% L-Anteil, bevorzugt jedoch nicht mehr als 95%. Ein besonders bevorzugter Bereich liegt bei 75 bis 85% L-Anteil im Copolymer.

Beträgt der Anteil an Poly-L-lactid in der Polymermischung über 85% sollte der Anteil an L-lactid im Copolymerisat nicht mehr als 90% betragen.

Bevorzugt sind ferner Formkörper aus einer Polymermischung enthaltend Poly(L-lactid-co-D,L-lactid) mit einem Anteil von 70 bis 95% L-lactid im Copolymerisat und Poly(D,L-lactid) mit einem Gewichtsanteil von 1 bis 85%, bevorzugt 5 bis 35, besonders bevorzugt 15 bis 35 Poly(D,L-lactid) in der Polymermischung und Formkörper aus Poly-L-lactid und Poly(D,L-Lactid) mit einem Gewichtsanteil von 5 bis 85% bevorzugt 15 bis 50%, besonders bevorzugt 15 bis 35% Poly(-D,L-Lactid) in der Polymermischung.

Es gibt verschiedene Methoden, die erfindungsgemäßen Mischungen aus Poly(L-lactid) und den oben beschriebenen Zusatzstoffen herzustellen. Zum einen können Mischungen beider Komponenten direkt in das Spritzgußverfahren eingesetzt werden, zum anderen kann die Mischung beider Komponenten zu einem Granulat verarbeitet werden, welches dann spritzgegossen wird.

Gegenstand der Erfindung ist somit auch die Herstellung von Formkörpern im Spritzgußprozeß, wobei Polylactid, wie z.B. Poly(L-lactid) oder ein Copolymer aus L-Lactid und D,L-Lactid mit einem Gehalt bis zu 30 %, besonders bevorzugt zwischen 5 und 15 % D,L-Lactid mit einem polymeren Zusatzstoff oder ein Granulat der Mischung aus beiden eingesetzt wird.

So entstehen Spritzlinge, die sowohl feste als auch zähe Eigenschaften zeigen. Besonders bemerkenswert ist, daß die Festigkeit der erfindungsgemäßen Polymermischungen signifikant erhöht ist und anforderungsgemäß über den Zeitraum von 8 Wochen auf hohem Niveau erhalten bleibt.

Die nachfolgenden Beispiele sollen die Erfindung näher erläutern.

Als Spritzgußmaschine wurde eine vollhydraulische Anker Demag Kolbenmaschine verwendet (Kolbendurchmesser 21 mm, Zuhaltekraft 150 KN). Die Spritzgußbedingungen für die verschiedenen Polymere und Polymermischungen sind in Tabelle 6 zusammengestellt. Als Werkzeugtemperatur wurde für alle Kunststoffvarianten 40 °C, als Einspritzgeschwindigkeit die maximal mögliche gewählt. Für die Festigkeitsuntersuchungen wurde eine Universalprüfmaschine vom Typ JJ Lloyds T 5002 verwendet. Die Form der im Zugversuch getesteten Probekörper entsprach jeweils der Probe Nr. 4 aus DIN 53455, wobei die Dicke der Proben 3 mm betrug.

Beispiele

Beispiel 1: Herstellung von Formkörpern aus Poly(L-lactid)

Poly(L-lactid)-Probestäbchen (2 x 3 x 25 mm) wurden durch Spritzgießen aus Granulat mit der inhärenten Viskosität 7,8 dl/g sowie durch zerspanende Bearbeitung aus Blockware (i.V. 7,9 dl/g) hergestellt. Die Biegefestigkeit dieser Proben wurde im Anlieferungszustand und nach Sterilisation sowie in Abhängigkeit von der Implantationszeit in Anlehnung an DIN 53452 ermittelt. Als Versuchstiere dienten Ratten. Die Ergebnisse sind in Tabelle 1 dargestellt.

7

Tabelle 1

Biegefestigkeit von Probekörpern aus Poly(L-lactid)
nach Implantation in Ratten (M/mm$^2$)

| Versuchsreihe | Werkstoff | Verarbeitungszustand |
|---|---|---|
| Nr. 1 | Poly(L-lactid)<br>(i.V. 7,8 dl/g) | gespritzt,<br>i.V. 3,08 dl/g<br>sterilisiert, amorph |
| Nr. 2 | Poly(L-lactid)<br>(i.V. 2,9 dl/g) | gespritzt,<br>i.V. 1,65 dl/g,<br>sterilisiert, amorph |
| Nr. 3 | Poly(L-lactid) | Blockpolymer,<br>i.V. 7,9 dl/g,<br>sterilisiert, kristallin |

Implantationszeit (Wochen)

| Vers.<br>reihe | O | 2 | 4 | 6 | 8 |
|---|---|---|---|---|---|
| Nr. 1 | 130,3 | 120,2 | 112,5 | 107,1 | 106,3 |
| Nr. 2 | 118,3 | 109,3 | 107,6 | 105,4 | 114,7 |
| Nr. 3 | 118,8 | 78,8 | 54,7 | 32,3 | 22,0 |

| Vers.<br>reihe | 12 | 20 | 24 | 32 |
|---|---|---|---|---|
| Nr. 1 | 99,6 | 65,1 | 51,3 | 19,2 |
| Nr. 2 | 124,0 | 77,5 | 68,0 | 30,2 |
| Nr. 3 | 15,5 | 9,8 | 7,4 | 4,6 |

Beispiel 2: Anfangsfestigkeit von spritzgegossenen Formkörpern aus Poly(L-lactid) mit niedriger inhärenter Viskosität

Aus Poly(L-lactid)-Granulat (i.V. 2,91 dl/g) wurden in einer Spritzgießmaschine Probekörper für den Zugversuch nach DIN 53455 (Prüfgeschwindigkeit: 10 mm/min) hergestellt. Die Eigenschaften dieser Probekörper zeigt Tab. 2 A. Die Ermittlung der Biegefestigkeit wurde in Beispiel 1 beschrieben.
In Tabelle 2 B sind Vergleichswerte für Probekörper aus Polymerblöcken angegeben.

**EP 0 401 844 B1**

Tabelle 2 A

| Mechanische Eigenschaften spritzgegossener Formkörper | |
|---|---|
| Inhärente Viskosität | 1,45 dl/g |
| Zugfestigkeit | 46,4 N/mm$^2$ |
| Bruchdehnung | 0 % |
| Biegefestigkeit | 118,3 N/mm$^2$ |

Tabelle 2 B

| Mechanische Eigenschaften von Formkörpern aus Polymerblöcken | | | | |
|---|---|---|---|---|
| Inhärente Viskosität | 7,9 | 7,4* | 3,7* | dl/g |
| Zugfestigkeit | | 58,2 | 28,8 | N/mm$^2$ |
| Biegefestigkeit | 118,8 | | | N/mm$^2$ |

\* aus EP 0108635

Beispiel 3: Formkörper aus Poly(L-lactid-co-D,L-lactid)

Granulat aus Poly(L-lactid-co-D,L-lactid) 90 : 10 (i.V. 7,0 dl/g) wurde zur Herstellung von spritzgegossenen Probekörpern für den Zugversuch gemäß DIN 53455 (Prüfgeschwindigkeit: 10 mm/min) verwendet. Die Eigenschaften dieser Probekörper sind in Tabelle 3 zusammengestellt.

Tabelle 3

| Inhärente Viskosität | 2,10 dl/g |
|---|---|
| Zugfestigkeit | 51,6 N/mm$^2$ |
| Bruchdehnung | 2,0 % |

Beispiel 4: Einfluß von niedermolekularen Weichmachern auf Poly(L-lactid)

In Poly(L-lactid) wurden 8,6 % Acetyltributylcitrat eingearbeitet. Die inhärente Viskosität des daraus hergestellten Granulats betrug 1,72 dl/g. Aus diesem Granulat wurden spritzgegossene Probekörper für den Zugversuch nach DIN 53455 (Prüfgeschwindigkeit : 10 mm/min) angefertigt.
In Tabelle 4 wurden die Eigenschaften zusammengestellt:

Tabelle 4

| Inhärente Viskosität | 1,40 dl/g |
|---|---|
| Zugfestigkeit | 46,2 N/mm$^2$ |
| Bruchdehnung | 3,0 % |

Bei einem analog hergestellten Poly(L-lactid) mit 10 % Butylbutyryllactat wurde eine Zugfestigkeit von ebenfalls 46,2 N/mm$^2$, aber eine unveränderte Bruchdehnung von 0 % gefunden. Der Zusatz von 1,5 % beziehungsweise 4,7 % Triethylcitrat führte gegenüber dem reinen Poly(L-lactid) zu verringerten Zugfestigkeiten von 14,2 N/mm$^2$ beziehungsweise 9,7 N/mm$^2$. Die Bruchdehnung betrug unverändert 0 %.

Beispiel 5: Einfluß hochmolekularer Feststoffe auf Poly(L-lactid)

Im Zugversuch gemäß DIN 53455 wurden gespritzte Probekörper aus Polymermischungen, bestehend aus Poly(L-lactid) und Poly(D,L-lactid) mit einer Prüfgeschwindigkeit von 10 mm/min getestet. Die mechanischen Eigenschaften in Abhängigkeit vom Mischungsverhältnis wurden in Tabelle 5 A zusammengestellt. Tabelle 5 B zeigt die Veränderung der mechanischen Eigenschaften nach Hydrolyse in Ringerlösung bei 37 °C. Auch Vergleichswerte für hydrolysierte Zugstäbe aus gespritztem Poly(L-lactid) sind angegeben.

Tabelle 5 A

| Zugversuch an gespritzten Probekörpern aus Poly(L-lactid) und Polymermischungen Poly(L-lactid)/Poly(D,L-lactid) (PLLA/PDLLA) (DIN 53455; Mittelwert x; n = 5) | | | |
|---|---|---|---|
| Werkstoff | Zugfestigkeit x [N/mm$^2$] | Bruchdehnung x [%] | $\eta^*$ dl/g |
| PLLA | 46,4 | 0 | 1,45 |
| PLLA/PDLLA 90/10 | 63,3 | 3,8 | 1,75 |
| PLLA/PDLLA 80/20 | 64,5 | 3,6 | 1,82 |
| PLLA/PDLLA 70/30 | 59,7 | 5,2 | 1,54 |
| PLLA/PDLLA 50/50 | 55,3 | 2,4 | 1,47 |
| PLLA/PDLLA 30/70 | 55,0 | 6,0 | 1,65 |
| PLLA = Poly(L-lactid) | | | |
| PDLLA = Poly(D,L-lactid) | | | |

*inhärente Viskosität

Tabelle 5 B

| Zugversuch an gespritzten Proben aus Polymermischungen Poly(L-lactid)/Poly(D,L-lactid) sowie Poly(L-lactid) nach Hydrolyse in Ringerlösung bei 37 °C (DIN 53455; Mittelwert x; n = 5) | | | | | |
|---|---|---|---|---|---|
| Werkstoff | | Hydrolysezeit in Wochen | | | |
| | | 0 | 4* | 8* | 12* |
| PLLA/PDLLA 90/10 | Zugfestigkeit (N/mm$^2$) x | 63,3 | 56,3 | 53,6 | 52,8 |
| | Bruchdehnung (%) x | 3,8 | 16,0 | 6,0 | 5,0 |
| PLLA/PDLLA 70/30 | Zugfestigkeit (N/mm$^2$) x | 59,7 | 50,7 | 49,0 | 46,9 |
| | Bruchdehnung (%) x | 5,2 | 9,3 | 9,0 | 6,5 |
| PLLA | Zugfestigkeit (N/mm$^2$) x | 46,4 | 37,6 | 35,4 | 29,0 |
| | Bruchdehnung (%) x | 0 | 0 | 0 | 0 |

* Proben wurden in wassergesättigtem Zustand gemessen.

Tabelle 6

Spritzgußbedingungen für Zugstäbe für verschiedene Polymere und Polymermischungen

| Polymer | Zylinder-temp. °C | Einspritz-zeit (s) | Einspritz-druck (bar) | Nach-druck (bar) | Nach-druck-zeit (s) | Kühl-zeit (s) |
|---|---|---|---|---|---|---|
| PLLA | 190 ° | 10 | 1120 | 600 | 1,5 | 15 |
| Poly(L-lactid-co-D, L-Lactid) 90/10 | 238 ° | 10 | 1110 | 600 | 1,5 | 15. |
| PLLA + 8,6 % - Acetyltributylcitrat | 185 ° | 10 | 1130 | 600 | 1,5 | 15 |
| PLLA + 10 % Butyl-butyryllactat | 185 ° | 10 | 1150 | 600 | 1,5 | 15 |
| PLLA + 1,5 % Tri-ethylcitrat | 180 ° | 10 | 1130 | 600 | 1,5 | 15 |
| PLLA + 4,7 Tri-ethylcitrat | 180 ° | 10 | 1130 | 600 | 1,5 | 15 |

| Polymer | Zylinder-temp. °C | Einspritz-zeit (s) | Einspritz-druck (bar) | Nach-druck (bar) | Nach-druck-zeit (s) | Kühl-zeit (s) |
|---|---|---|---|---|---|---|
| PLLA/PDLLA 90/10 | 190 ° | 10 | 1150 | 600 | 1,5 | 15 |
| PLLA/PDLLA 80/20 | 183 ° | 10 | 1150 | 600 | 1,5 | 15 |
| PLLA/PDLLA 70/30 | 175 ° | 10 | 1120 | 600 | 1,5 | 15 |
| PLLA/PDLLA 50/50 | 180 ° | 10 | 1150 | 600 | 1,5 | 15 |
| PLLA/PDLLA 30/70 | 175 ° | 10 | 1150 | 600 | 1,5 | 15 |

PLLA = Poly(L-lactid)
PDLLA= Poly(D,L-lactid)

**Patentansprüche**

1.  Resorbierbarer Formkörper auf der Basis von Poly(lactid), oder davon abgeleitete Copolyinerisate dadurch gekennzeichnet, daß er einen hochmolekularen Zusatzstoff aus einem resorbierbaren Polyester in einer wirksamen Menge zur Erhöhung der Zugfestigkeit und der Bruchdehnung enthält, wobei Poly [p-dioxanon] als Zusatzstoff ausgenommen ist und der Glycolidgehalt der Gesamtzusammensetzung weniger als 65 Gew.-% beträgt.

2.  Formkörper nach Anspruch 1, dadurch gekennzeichnet, daß er aus Poly(L-lactid), Poly(D-lactid) oder davon abgeleiteten Copolymerisaten mit anderen Comonomeren in Form copolymerisierbarer cyclischer Ester besteht, wobei der Comonomeranteil nicht mehr als 30 Gew.-%, bevorzugt nicht mehr als 15 Gew.-% beträgt.

3.  Formkörper nach Anspruch 2, dadurch gekennzeichnet, daß das Comonomer D,L-Lactid, meso-Lactid, Glycolid, Trimethylencarbonat oder ein Lacton der $\beta$-Hydroxybuttersäure und/oder $\beta$-Hydroxyvaleriansäure ist.

4.  Resorbierbarer Formkörper nach einem der Ansprüche 1 bis 3, dadurch gekennzeichnet, daß er Poly(L-lactid) oder Poly(L-lactid-co-D,L-lactid) enthält.

5.  Resorbierbarer Formkörper nach einem der Ansprüche 1 bis 4, dadurch gekennzeichnet, daß der hochmolekulare Zusatzstoff Poly(D,L-lactid), Poly(D-lactid), Poly(meso-lactid), Polyglycolid, Polytrimethylencarbonat, Polycaprolacton oder deren Gemisch ist.

6.  Resorbierbarer Formkörper nach einem der Ansprüche 1 bis 5, dadurch gekennzeichnet, daß der Zusatzstoff ein Copolymerisat ist ausgewählt aus der Gruppe:
    Poly(L-lactid-co-D,L-lactid)

EP 0 401 844 B1

Poly(L-lactid-co-meso-lactid)
Poly(L-lactid-co-glycolid)
Poly(L-lactid-co-trimethylencarbonat)
Poly(L-lactid-co-$\epsilon$-caprolacton)
Poly(D,L-lactid-co-meso-lactid)
Poly(D,L-lactid-co-glycolid)
Poly(D,L-lactid-co-trimethylencarbonat)
Poly(D,L-lactid-co-$\epsilon$-caprolacton)
Poly(meso-lactid-co-glycolid)
Poly(meso-lactid-co-trimethgylencarbonat)
Poly(meso-lactid-co-$\epsilon$-caprolacton)
Poly(glycolid-co-trimethylencarbonat)
Poly(glycolid-co-$\epsilon$-caprolacton)

7. Resorbierbarer Formkörper nach einem der Ansprüche 1 bis 6, dadurch gekennzeichnet, daß er 1 bis 85 Gew.-% des Zusatzstoffes enthält.

8. Resorbierbarer Formkörper nach einem der Ansprüche 1 bis 7, dadurch gekennzeichnet, daß er eine Bruchdehnung von mindestens 2 % im Ansgangszustand und unter physiologischen Bedingungen für die Dauer von 8 Wochen aufweist.

9. Resorbierbarer Formkörper nach einem der Ansprüche 1 bis 8, dadurch gekennzeichnet, daß er durch Spritzgießverfahren herstellbar ist.

10. Verwendung von hochmolekularen resorbierbaren Polyestern als Zusatzstoff in einer wirksamen Menge zur Erhöhung der Zugfestigkeit und der Bruchdehnung von Formkörpern auf der Basis von Polylactid.

11. Verwendung nach Anspruch 10, dadurch gekennzeichnet, daß als Zusatzstoff Poly(D,L-lactid), Poly(D-lactid), Poly(meso-lactid), Polyglycolid, Poly(trimethylencarbonat), Poly($\epsilon$-caprolacton) oder deren Gemische verwendet werden.

**Claims**

1. Resorbable mouldings based on poly(lactide) or copolymers derived therefrom, characterised in that they contain a high molecular weight additive consisting of a resorbable polyester in an effective amount in order to increase the tensile strength and elongation at breakage, poly[p-dioxanone] being excluded as the additive and the glycolide content of the entire composition being less than 65 wt. %.

2. Mouldings according to claim 1, characterised in that they comprise poly(L-lactide), poly(D-lactide) or copolymers derived therefrom with other comonomers in the form of copolymerisable cyclic esters, the proportion of comonomer being not more than 30% by weight, preferably not more than 15% by weight.

3. Mouldings according to claim 2, characterised in that the comonomer is D,L-lactide, meso-lactide, glycolide, trimethylenecarbonate or a lactone of $\beta$-hydroxybutyric acid and/or $\beta$-hydroxyvaleric acid.

4. Resorbable mouldings according to one of claims 1 to 3, characterised in that they comprise poly(L-lactide) or poly(L-lactide-co-D,L-lactide).

5. Resorbable mouldings according to one of claims 1 to 4, characterised in that the high molecular weight additive is poly(D,L-lactide), poly(D-lactide), poly(meso-lactide) polyglycolide, polytrimethylene carbonate, polycaprolactone or a mixture thereof.

6. Resorbable mouldings according to one of claims 1 to 5, characterised in that the additive is a copolymer selected from the group:
poly(L-lactide-co-D,L-lactide)
poly(L-lactide-co-meso-lactide)
poly(L-lactide-co-glycolide)

13

poly(L-lactide-co-trimethylene carbonate)
poly(L-lactide-co-$\epsilon$-caprolactone)
poly(D, L-lactide-co-meso-lactide)
poly(D, L-lactide-co-glycolide)
poly(D,L-lactide-co-trimethylene carbonate)
poly(D, L-lactide-co-$\epsilon$-caprolactone)
poly(meso-lactide-co-glycolide)
poly(meso-lactide-co-trimethylene carbonate)
poly(meso-lactide-co-$\epsilon$-caprolactone)
poly(glycolide-co-trimethylene carbonate)
poly(glycolide-co-$\epsilon$-caprolactone)

7. Resorbable mouldings according to one of claims 1 to 6, characterised in that they comprise 1 to 85% by weight of the additive.

8. Resorbable mouldings according to one of claims 1 to 7, characterised in that they have an elongation at breakage of at least 2% in the initial state and under physiological conditions for a period of 8 weeks.

9. Resorbable mouldings according to one of claims 1 to 8, characterised in that they can be produced by injection moulding.

10. Use of high molecular resorbable polyesters as additive in an effective quantity for increasing the tensile strength and elongation at breakage of mouldings based on polylactide.

11. Use according to claim 10, characterised in that the additive used is poly(D,L-lactide), poly(D-lactide), poly(meso-lactide), poly(glycolide), poly(trimethylene carbonate), poly($\epsilon$-caprolactone) or mixtures thereof.

**Revendications**

1. Article moulé résorbable à base de poly(lactide) ou de produits de copolymérisation qui en dérivent, caractérisé en ce qu'il contient un additif à haute masse moléculaire constitué par un polyester résorbable en une quantité efficace pour augmenter la résistance à la traction et l'allongement à la rupture, la poly[p-dioxanone] étant exclue comme additif et la teneur en glycolide de la composition totale étant inférieure à 65% en masse.

2. Article moulé selon la revendication 1, caractérisé en ce qu'il consiste en poly(L-lactide), en poly(D-lactide) ou en produits de copolymérisation qui en dérivent avec d'autres comonomères sous forme d'esters cycliques copolymérisables, la proportion de comonomère ne dépassant pas 30% en masse, de préférence ne dépassant pas 15% en masse.

3. Article moulé selon la revendication 2, caractérisé en ce que le comonomère est le D,L-lactide, le méso-lactide, le glycolide, le carbonate de triméthylène ou une lactone de l'acide $\beta$-hydroxybutyrique et/ou de l'acide $\beta$-hydroxyvalérique.

4. Article moulé résorbable selon l'une des revendications 1 à 3, caractérisé en ce qu'il contient du poly-(L-lactide) ou un copolymère(L-lactide-D,L-lactide).

5. Article moulé résorbable selon l'une des revendications 1 à 4, caractérisé en ce que l'additif à haute masse moléculaire est le poly(D,L-lactide), le poly(D-lactide), le poly(méso-lactide), le polyglycolide, le poly(carbonate de triméthylène), la polycaprolactone ou leur mélange.

6. Article moulé résorbable selon l'une des revendications 1 à 5, caractérisé en ce que l'additif est un produit de copolymérisation choisi dans le groupe:
copolymère (L-lactide-D,L-lactide)
copolymère (L-lactide-méso-lactide)
copolymère (L-lactide-glycolide)
copolymère (L-lactide-carbonate de triméthylène)

14

copolymère (L-lactide-$\epsilon$-caprolactone)
copolymère (D,L-lactide-méso-lactide)
copolymère (D,L-lactide-glycolide)
copolymère (D,L-lactide-carbonate de triméthylène)
copolymère (D,L-lactide-$\epsilon$-caprolactone)
copolymère (méso-lactide-glycolide)
copolymère (méso-lactide-carbonate de triméthylène)
copolymère (méso-lactide-$\epsilon$-caprolactone)
copolymère (glycolide-carbonate de triméthylène)
copolymère (glycolide-$\epsilon$-caprolactone).

7. Article moulé résorbable selon l'une des revendications 1 à 6, caractérisé en ce qu'il contient 1 à 85% en masse d'additif.

8. Article moulé résorbable selon l'une des revendications 1 à 7, caractérisé en ce qu'il présente un allongement à la rupture d'au moins 2% à l'état initial et dans des conditions physiologiques pendant une durée de 8 semaines.

9. Article moulé résorbable selon l'une des revendications 1 à 8, caractérisé en ce qu'il peut être fabriqué par un procédé de moulage par injection.

10. Utilisation de polyesters résorbables à haute masse moléculaire comme additif en une quantité efficace pour augmenter la résistance à la traction et l'allongement à la rupture d'articles moulés à base de polylactide.

11. Utilisation selon la revendication 10, caractérisée en ce que l'on utilise comme additif le poly(D,L-lactide), le poly(D-lactide), le poly(méso-lactide), le polyglycolide, le poly(carbonate de triméthylène), la poly($\epsilon$-caprolactone) ou leurs mélanges.